# EUROPÄISCHE PATENTSCHRIFT

(11) **EP 0 348 464 B1**
(45) Veröffentlichungstag und Bekanntmachung des Hinweises auf die Patenterteilung: **31.03.1993**
(21) Anmeldenummer: 89900203.4
(22) Anmeldetag: 21.09.1988
(51) Int. Cl.: C07C 50/18

(54) **2-NEOPENTYLANTHRACHINON**
2-NEOPENTYLANTHRAQUINONE
2-NEOPENTYLANTHRAQUINONE

(30) Priorität: 23.09.1987 DE 3732015
(43) Veröffentlichungstag der Anmeldung: 03.01.1990
(73) Patentinhaber: BASF Aktiengesellschaft, 67063 Ludwigshafen (DE)
(72) Erfinder: EGGERSDORFER, Manfred, D-6710 Frankenthal (DE); HENKELMANN, Jochen, 6800 Mannheim 1 (DE); GROSCH, Walter, D-6803 Edingen-Neckarhausen (DE)
(86) Internationale Anmeldenummer: EP8800854
(87) Internationale Veröffentlichungsnummer: WO8902886

(56) Entgegenhaltungen:
- EP-A- 0 025 620
- DE-B- 1 157 595

## Beschreibung

Die vorliegende Erfindung betrifft die neue Verbindung 2-Neopentylanthrachinon mit der Formel

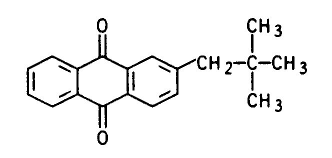

Weiterhin betrifft die Erfindung die Herstellung von 2-Neopentylanthrachinon durch Freidel-Crafts-Acylierung von Neopentylglykol mit Phthalsäureanhydrid und anschließende Cyclisierung der erhaltenen 2-(4-Neopentylbenzoyl)-benzoesäure in Gegenwart starker, wasserfreier Säuren.

Fernerhin betrifft die Erfindung die Verwendung von 2-Neopentylanthrachinon als Katalysator bei der Wasserstoffperoxidherstellung.

Das technisch wichtigste verfahren zur Wasserstoffperoxidherstellung ist das Anthrachinon-Verfahren. Für dieses Verfahren werden 2-Alkylanthrachinone als Katalysatoren benötigt.

In dem als Kreizprozeß durchgeführten Verfahren wird das gelöste 2-Alkylanthrachinon katalytisch zum 2-Alkylanthrahydrochinon hydriert, das nach Abrennung des Hydrierkatalysators mit Sauerstoff wieder zum 2-Alkylanthrachinon oxidiert wird. Bei diesem zweiten Schritt wird Wasserstoffperoxid gebildet, das mit Wasser aus der Arbeitslösung extrahiert wird. Das rückgebildete 2-Alkylanthrachinon wird in den Kreislauf zurückgeführt. Eine genauere Beschreibung des Prozesses findet man beizpielsweise in Ullman, Bd. 17, s. 691 ff (4. Auflage, Weinheim, New York, 1979).

Als Katalysatoren in diesem Prozeß wurden in der Patentliteratur verschiedene 2-Alkylanthrachinone vorgeschlagen, beispielsweise 2-Methyl-, 2-Ethyl- und 2-Propylanthrachinon (US 2 158 525), 2-Isopropyl-, 2-sek.-Butylanthrachinon und 2-tert.-Butylanthrachinon (US 2 935 381) und "2-Amylanthrachinon" (DE-AS 11 12 051, US-PS 3 041 143).

Bei "2-Amylanthrachinon" handelt es sich um ein Gemisch aus etwa gleichen Teilen 2-tert.-Pentylanthrachinon und 2-(3-Methylbut-2-yl)-anthrachinon. Dieses Gemisch resultiert aus der ersten Stufe der Anthrachinonsynthese, nämlich der Friedel-Crafts-Acylierung von tert.-Pentylbenzol mit Phthalsäureanhydrid, bei der etwa die Hälfte der tert.-Pentylgruppen zu 3-methylbut-2-yl isomerisieren.

2-Amylanthrachinon-Gemische mit hohen Anteilen an tert.-Amylanthrachinon haben sich als besonders vorteilhaft für die Wasserstoffperoxidherstellung erweisen (DE-OS 20 13 299).

Die Isomerisierung der tert.-Amylgruppe unter den Synthesebedingungen kann zwar teilweise unterdrückt werden; allerdings sind dazu zusätzliche Maßnahmen erforderlich (DE-PS 2 720 294). Diese zusätzlichen Maßnahmen erfordern z.T. aufwendige technische Apparaturen und ergeben unbefriedigende Ausbeuten.

Wichtig für eine lange Lebensdauer des 2-Alkylanthrachinon/2-Alkylanthrahydrochinon-Systems ist eine hohe Stabilität des 2-Alkylanthrachinons. Ferner ist für eine gute Raum/Zeit-Ausbeute des Wasserstoffperoxid-Prozesses eine hohe Löslichkeit des 2-Alkylanthrachinon/2-Alkylanthrahydrochinon-Systems in den bei der H₂O₂-Herstellung verwendeten Lösungsmittelsystemen günstig.

Die Amylanthrachinon-Gemische haben aber den Nachteil, daß nur die tert.-Amylverbindung gute Stabilität zeigt, während 3-methylbut-2-yl anthrachinon unter den Reaktionsbedingungen der H₂0₂-Synthese oxidativ angegriffen wird.

Es bestand daher die Aufgabe für die Wasserstoffperoxid-Synthese einen Katalysator zu finden, der eine hohe Löslichkeit in der Chinon- und Hydrochinonform in der Arbeitslösung zeigt, der unter den Bedingungen der H₂0₂-Synthese gute Stabilität zeigt und zudem einfach und auf wirtschaftlichem Weg hergestellt werden kann.

Demgemäß wurde die neue Verbindung 2-Neopentylanthrachinon der Formel 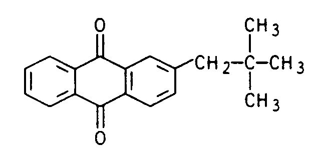
gefunden. Weiterhin wurde ein Verfahren zur Herstellung von 2-Neopentylanthrachinon gefunden, dadurch gekennzeichnet, daß man Neopentylbenzol mit Phthalsäureanhydrid nach Friedel-Crafts zu 2-(4-Neopentylbenzoyl)-benzoesäure acyliert und diese in Gegenwart von starken wasserfreien Säuren zum 2-Neopentylanthrachinon cyclisiert. Fernerhin wurde gefunden, daß 2-Neopentylanthrachinon ein Katalysator zur Herstellung von Wasserstoffperoxid ist.

2-Neopentylanthrachinon wird aus Neopentylbenzol und Phthalsäureanhydrid in guten Ausbeuten erhalten, ohne daß eine Isomerisierung oder ein Abbau der Neopentylgruppe stattfinden.

Bei der Synthese anderer 2-Alkylanthrachinone führt ein Abbau der Alkylgruppe unter den Synthesebedingungen oft zur Bildung unerwünschter Nebenprodukte wie beispielsweise Anthrachinon, 2-Methylanthrachinon und 2-Isopropylanthrachinon, die in der H₂0₂-Synthese stören und daher in aufwendigen Reinigungsschritten abgetrennt werden müssen.

Da sich bei der Synthese von 2-Neopentylanthrachinon keine derartigen Nebenprodukte bilden, kann auf diese aufwendige Reinigung verzichtet werden.

Nach dem erfindungsgemäßen Verfaren wird Neopentylbenzol (1) im 1. Schritt mit Phthalsäureanhydrid (2) unter Friedel-Crafts-Bedingungen zur 2-(4-Neopentylbenzoyl)-benzoesäure (3) acyliert.

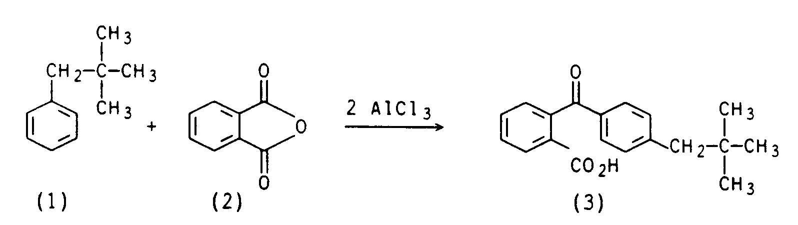

Neopentylbenzol ist eine bekannte Verbindung und ist in J. Chem. Soc. (C) 18, 2505-6 (1969) beschrieben.

Als Freidel-Crafts-Katalysatoren können für diese Reaktion die an sich üblichen Verbindungen wie FeCl₃, BF₃, ZnCl₂, TiCl₄ verwendet werden. Besonders geeignet sind Aluminiumhalogenide, bevorzugt Aluminiumbromid und insbesondere Aluminiumchlorid. Auch könner Gemische dieser Katalysatoren verwendet werden.

Die für Friedel-Crafts-Reaktionen üblicherweise verwendeten Katalysatormengen können auch beim erfindungsgemäßen Verfahren eingesetzt werden, wobei zweckmäßig 2 mol Katalysator bezogen auf 1 mol Phthalsäureanhydrid verwendet werden. Es kann jedoch auch ein Überschuß an Katalysator, beispielsweise 2,1 bis 3,5 mol Katalysator bezogen auf 1 mol Phthalsäureanhydrid, angewandt werden.

Zweckmäßigerweise werden stöchiometrische Mengen an Neopentylbenzol und Phthalsäureanhydrid verwendet. Es ist aber auch möglich, eine der beiden Komponenten gegenüber der anderen im Überschuß einzusetzen. So kann man beispielsweise 1 bis 1,5 mol Neopentylbenzol je Mol Phthalsäureanhydrid verwenden oder umgekehrt.

Man führt die Reaktion ohne oder vorteilhaft in Gegenwart eines Lösungsmittels aus, wobei als Lösungsmittel z.B. Chlorbenzol, Dichlorbenzol, Trichlorbenzol, 1,2-Dichlorethan, Schwefelkohlenstoff, Nitromethan oder Nitrobenzol in Betracht kommen. Die Lösungsmittelmenge ist variabel, im allgemeinen können 200 bis 1000 g Lösungsmittel je Mol Neopentylbenzol verwendet werden.

Die Durchführung der Reaktion erfolgt zweckmäßig in der Weise, daß man die Ausgangsstoffe bei Temperaturen von -20 bis 100, vorzugsweise 0 bis 60, insbesondere 10 bis 40°C und bei erhöhtem oder vermindertem Druck, vorzugsweise bei Normaldruck umsetzt.

Zweckmäßigerweise legt man das Phthalsäureanhydrid zusammen mit dem Lösungsmittel vor und gibt den Friedel-Crafts-Katalysator und anschließend das Neopentylbenzol hinzu, jedoch ist auch die umgekehrte Reinhenfolge möglich.

Die Aufarbeitung des Reaktionsgemisches und die Isolierung des Produktes erfolgt im allgemeinen in üblicher Weise, indem man das Reaktionsgemisch auf Wasser und/oder Eis gießt und nach Abrennung der wässrigen Phase die 2-(4-Neopentylbenzoyl)-benzoesäure aus der organischen Phase isoliert.

Die 2-(4-Neopentylbenzoyl)-benzoesäure (3) wird vorteilhaft in Gegenwart starker, wasserfreier Säuren zum 2-Neopentylanthrachinon cyclisiert (s. Gleichung).

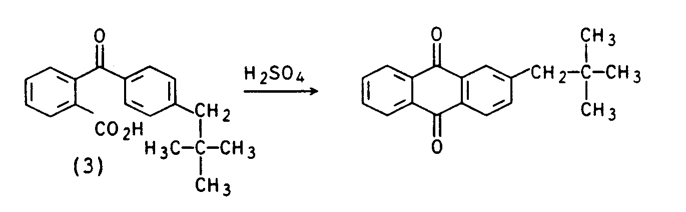

Die Cyclisierung der 2-(4-Neopentylbenzoyl)-benzoesäure erfolgt zweckmäßig in an sich bekannter Weise, z.B. nach den in Houben-Weyl Bd. 7/3c, S. 35 bis 39 beschriebenen Methoden, vorteilhaft mit 95 %iger bis 105 %iger Schwefelsäure (= 5er Oleum), bevorzugt mit 100 %iger Schwefelsäure.

Zweckmäßigerweise legt man die Schwefelsäure vor und gibt hierzu die 2-(4-Neopentylbenzoyl)-benzoesäure. Die Menge an Schwefelsäure beträgt dabei im allgemeinen 0,5 bis 10, vorzugsweise 0,8 bis 1,0 kg pro kg der 2-(4-Neopentylbenzoyl)-benzoesäure. Die Reaktionsmischung wird dann im allegemeinen 1 bis 10 Stunden bei 20 bis 220°C, bevorzugt jedoch bei 60 bis 90°C gerührt. Die Reaktion kann unter Atmosphärendruck, unter Druck oder unter vermindertem Druck vorgenommen werden.

Die Reaktion wird abgebrochen, indem man das Reaktionsgemisch z.B. in Eis und/oder Wasser einträgt. Das Produkt kann dann durch Extraktion mit einem geeigneten Lösungsmittel aus dem Reaktionsgemisch abgetrennt werden. Als Lösungsmittel eignen sich z.B. aromatische Kohlenwasserstoffe wie Benzol, Toluol und Xylol oder chlorierte Kohlenwasserstoffe wie Chlorbenzol, Dichlormethan, Dichlorethan oder Trichlorethan.

Die Lösung des 2-Neopentylanthrachinons wird zweckmäßig mit verdünnter Natronlauge und nachfolgend mit Wasser neutral gewaschen. Anschließend wird das Produkt nach Abtrennung des Lösungsmittels isoliert.

Die Aufarbeitung kann aber auch ohne Lösungsmittel erfolgen. Bei dieser Arbeitsweise geht man vorteilhaft so vor, daß man die Reaktion durch Eintragen des Reaktionsgemisches in Wasser und/oder Eis abbricht und das ausgefallen Reaktionsprodukt abfiltriert. Nachdem man das Reaktionsprodukt neutral gewaschen hat, isoliert man daraus das 2-Neopentylanthrachinon vorzugsweise durch Vakuumdestillation. Dieses Vorgehen hat nicht nur einen geringeren Lösungsmittelverbrauch zur Folge, sondern auch eine geringere Belastung der Umwelt durch Lösungsmittelemissionen.

2-Neopentylanthrachinon eignet sich in hervorragender Weise als Katalysator zur Herstellung von Wasserstoffperoxid nach dem Anthrachinon-Verfahren (Ullmans Enzyclopädie der Technischen Chemie, Bd. 17, S. 691 ff, 4. Auflage, Weinheim, 1979). Ebenso kann es aber auch vorteilhaft in Mischungen mit anderen Anthrachinonen, wie 2-Methylanthrachinon, 2-Ethylanthrachinon, 2-Propylanthrachinon, 2-Isopropylanthrachinon, 2-sek.-Butylanthrachinon, 2-tert.-Butylanthrachinon und 2-Amylanthrachinon als Katalysator eingesetzt werden. Dabei kann das Mischungsverhältnis in weiten Grenzen varrieren, da es einerseits von der Art der zugemischten 2-Alkylanthrachinone, andererseits von den jeweiligen Verfahrensbedingungen (Temperatur, Lösungsmittel usw.) in den jeweiligen Anlagen zur Wasserstoffperoxidherstellung abhängt.

### Beispiel

74 g (0,5 mol) Phthalsäureanhydrid wurden in 250 ml o-Dichlorbenzol gelöst. 128 g (1,05 mol) AlCl₃ wurden in Portionen bei 15 bis 20°C zugegeben. Danach wurden innerhalb von 3 Stunden 74 g (0,5 mol) Neopentylbenzol bei 20°C zugetropft. Anschließend wurde 1 Stunde bei 40°C nachgerührt.

Nach beendeter Reaktion wurde der Reaktionsaustrag auf ein Gemisch von 1 l Wasser mit 300 g Eis und 30 ml H₂S0₄ (konz.) gegossen. Die organische Phase wurde mit verdünnter Natronlauge neutral gewaschen. Anschließend wurde die Neopentylbenzoyl-benzoesäure aus der wässrigen Phase mit Schwefelsäure ausgefällt und getrocknet. Man erhielt 140,8 g (95 %) 2-(4-Neopentylbenzoyl)-benzoesäure (Fp. 154,2°C).

90 g (0,3 mol) dieser 2-(4-Neopentylbenzoyl)-benzoesäure wurden bei 60°C in 900 g 100-prozentige Schwefelsäure eingetragen und anschließend 4 Stunden bei 85°C gerührt. Danach wurde die Reaktionsmischung in eine Mischung aus 1500 g Wasser und 500 g Eis eingetragen und unter Rühren abgekühlt. Die sich abscheidenden Kristalle von Neopentylanthrachinon wurden abfiltriert und mit verd. Natronlauge und Wasser neutralgewaschen, getrocknet und bei 240°C/0,5 mbar destilliert. Man erhielt 75,3 g (89 %) 2-Neopentylanthrachinon (Fp. 93,8°C).

## Patentansprüche

1. 2-Neopentylanthrachinon der Formel

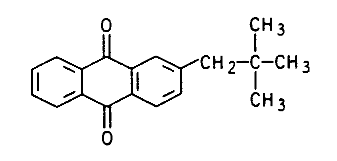

2. Verfahren zur Herstellung von 2-Neopentylanthrachinon, dadurch gekennzeichnet, daß man Neopentylbenzol mit Phthalsäureanhydrid nach Friedel-Crafts zur 2-(4-Neopentylbenzoyl)-benzoesäure acyliert und diese in Gegenwart von starken wasserfreien Säuren zum 2-Neopentylanthrachinon cyclisiert.

3. Verfahren nach Anspruch 2, dadurch gekennzeichnet, daß man die Cyclisierung mit 95 bis 105 %iger Schwefelsäure bei 20 bis 220°C durchführt.

4. Verwendung von 2-Neopentylanthrachinon als Katalysator für die Herstellung von Wasserstoffperoxid.

5. Verwendung von Mischungen von 2-Neopentylanthrachinon mit anderen Anthrachinonen als Katalysatoren zur Herstellung von Wasserstoffperoxid.

## Claims

1. A 2-neopentylanthraquinone of the formula

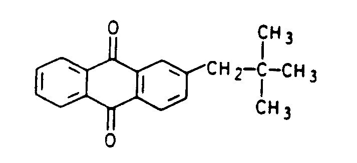

2. A process for the preparation of 2-neopentylanthraquinone, wherein neopentylbenzene is acylated with phthalic anhydride by a Friedel-Crafts reaction to give 2-(4-neopentylbenzoyl)-benzoic acid and the latter is cyclized in the presence of a strong anhydrous acid to give 2-neopentylanthraquinone.

3. A process as claimed in claim 2, wherein the cyclization is carried out with from 95 to 105% strength sulfuric acid at from 20 to 220°C.

4. Use of 2-neopentylanthraquinone as a catalyst for the preparation of hydrogen peroxide.

5. Use of a mixture of 2-neopentylanthraquinone with other anthraquinones as a catalyst for the preparation of hydrogen peroxide.

## Revendications

1. 2-Néopentylanthraquinone de formule

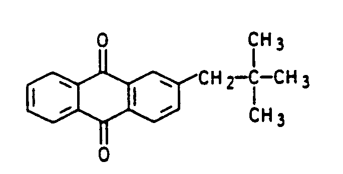

2. Procédé de préparation de 2-néopentylanthraquinone, caractérisé en ce qu'on acyle du néopentylbenzène par de l'anhydride phtalique en une réaction de Friedel et Crafts pour obtenir de l'acide 2-(4-néopentylbenzoyl)benzoïque et on cyclise celui-ci en présence d'acides forts anhydres pour obtenir la 2-néopentylanthraquinone.

3. Procédé selon la revendication 2, caractérisé en ce qu'on effectue la cyclisation à une température de 20 à 220°C avec de l'acide sulfurique à 95-105%.

4. Utilisation de 2-néopentylanthraquinone comme catalyseur pour la préparation de peroxyde d'hydrogène.

5. Utilisation de mélanges de 2-néopentylanthraquinone avec d'autres anthraquinones comme catalyseurs pour la préparation de peroxyde d'hydrogène.
